# EUROPEAN PATENT SPECIFICATION

(11) **EP 3 718 593 B1**
(45) Date of publication and mention of the grant of the patent: **14.12.2022**
(21) Application number: 19166778.1
(22) Date of filing: 02.04.2019
(51) Int. Cl.: A61M 25/10, A61B 17/12, A61F 2/00

(54) **TRANSURETHRAL CATHETER DEVICE FOR BLEEDING CONTROL IN PELVIC FRACTURES**
TRANSURETHRALE KATHETERVORRICHTUNG ZUR BLUTUNGSKONTROLLE BEI BECKENFRAKTUREN
DISPOSITIF DE CATHÉTER TRANSURÉTRAL POUR CONTRÔLE DU SAIGNEMENT DANS DES FRACTURES DU BASSIN

(43) Date of publication of application: 07.10.2020
(73) Proprietor: Universität Regensburg - Universitätsklinikum, 93053 Regensburg (DE)
(72) Inventor: Kerschbaum, Maximilian, 93055 Regensburg (DE)
(74) Representative: Lucke, Andreas

(56) References cited:
- DE-A1- 3 425 437
- DE-A1- 3 428 536
- US-A1- 2017 325 927

## Description

### FIELD OF THE INVENTION

The present invention is in the field of medical technology. In particular, the invention relates to a transurethral catheter device for bleeding control in pelvic fractures.

### BACKGROUND

Blood loss from internal bleeding constitutes a major challenge in the treatment of patients with pelvic fractures. The stability of the pelvis may be reduced due to the fracture, which may lead to an increase in the intrapelvic volume and may thereby complicate controlling the bleeding, see e.g. M. C. Moss and M. D. Bircher, Injury 27, Suppl 1:S-A21-3 (1996*).* During emergency treatment of patients, the main goal is thus to reduce the intrapelvic volume to minimize the available volume and to increase the pressure on the wound in order to stop the bleeding, see e.g. D. Köhler et al., J. Trauma 71, 585 (2011*).*

For this, a number of methods are currently being used. These include external compression, e.g. by placing a belt around the patient's pelvis, as well as a plurality of surgical methods to reduce the intrapelvic volume, e.g. by inserting a tamponade in the intrapelvic volume. External compression may often not be sufficient to control the bleeding. Surgical methods, on the other hand, require a high level of expertise and special equipment and in general cannot be performed by first responders. In some cases, it may not be possible to reach an appropriately equipped hospital in time, resulting in a considerable mortality rate among patients with internal bleeding in combination with a pelvic fracture. For stopping bleeding after prostate operations, DE3425437 discloses a catheter suitable to be placed in the sphincter.

### SUMMARY OF THE INVENTION

The object of the invention is thus to provide a device that facilitates an effective emergency treatment of patients with internal bleeding, particularly by first responders.

This object is met by a transurethral catheter device according to claim 1. Embodiments of the present invention are detailed in the dependent claims.

The transurethral catheter device for bleeding control in pelvic fractures comprises an expandable body assembly with at least one expandable body. The expandable body assembly comprises a proximal portion, a center portion and a distal portion and is configured to be expanded from a collapsed state to an expanded state by expanding the at least one expandable body. In the collapsed state, the expandable body assembly is configured to be inserted into the urinary bladder of a patient via the urethra. In the expanded state, the proximal portion has a length l₁ and a width w₁, the center portion has a length l₂ and a width w2, the distal portion has a length l₃ and a width w₃, and the expandable body assembly has a total length l. A displacement volume V of the expandable body assembly in the expanded state is larger than 0.5 liters. Furthermore, the width w₂ is smaller than 50% of the width w₃, the width w₁ is larger than a width *w̃*₁ of the proximal portion in the collapsed state and the width w₃ is larger than a width *w̃*₃ of the distal portion in the collapsed state. The length l₁ is smaller than 25% of the total length l. This shape of the expandable body assembly allows for arranging the expandable body assembly in the expanded state in the urinary bladder such that the proximal portion is adjacent to the internal urethral orifice of the urinary bladder and the center portion is adjacent to the ureter orifices of the urinary bladder, but not in contact with the ureter orifices.

The catheter device may for example be used for the treatment of internal bleeding in patients with a pelvic fracture, e.g. by inserting the expandable body assembly in the collapsed state into the urinary bladder of the patient via the urethra and subsequently expanding the expandable body assembly to the expanded state. The displacement volume of the expandable body assembly in the expanded state is larger than in the collapsed state. Thereby, the volume of the urinary bladder may be increased, thus reducing the intrapelvic volume and increasing the pressure in the abdomen. This may help to stop the internal bleeding. Inserting the expandable body assembly into the urinary bladder may not require additional equipment and may thus already be performed by first responders. Furthermore, urinary catheterization is often performed as a standard procedure in the treatment of patients with severe injuries and hence the catheter device according to the invention may be employed without complicating the treatment. In addition, the catheter device may be used in combination with known methods, e.g. external compression of the pelvis.

The expandable body assembly comprises at least one expandable body that is configured to be expanded to increase the displacement volume of the expandable body assembly. The displacement volume is defined as the volume of an incompressible fluid that is displaced by the expandable body assembly when fully immersing the expandable body assembly in the fluid. In the expanded state, the proximal portion, the center portion and the distal portion of the expandable body assembly are arranged along a longitudinal axis of the expandable body assembly such that the center portion is located between the proximal portion and the distal portion. The longitudinal axis extends from the proximal portion to the distal portion and may e.g. be defined as the axis around which the expandable body assembly has the smallest moment of inertia in the expanded state. The length of a portion is defined as the largest extent of the respective portion in the direction of the longitudinal axis. Accordingly, the total length of the expandable body assembly is the largest extent of the expandable body assembly in the direction of the longitudinal axis. The width of a portion is defined as the largest radially averaged width of the respective portion perpendicular to the longitudinal axis, wherein radial averaging of the width corresponds to taking the average of the width in a plane perpendicular to the longitudinal axis. Correspondingly, the total width w of the expandable body assembly is the largest width of widths of the individual portions.

In the collapsed state, the proximal portion has a length *l̃*₁ and the width *w̃*₁, the center portion a length *l̃*₂ and a width *w̃*₂, the distal portion a length *l̃*₃ and the width *w̃*₃, and the expandable body assembly a total length *l̃* and a total width *w̃*, wherein the lengths and widths may differ from the respective values in the expanded state. To facilitate inserting the expandable body assembly into the urinary bladder, the total width *w̃* may be less than 10 mm, preferably less than 5 mm. Preferably, the expandable body assembly comprises or consists of flexible materials.

The transurethral catheter device may further comprise a catheter tube assembly, which may e.g. be attached to or connected to the proximal portion and may extend outwards from the expandable body assembly, e.g. in proximal direction. Alternatively, the catheter device may comprise a connector configured to receive an external catheter tube assembly, wherein the connector may for example be arranged in the proximal portion. The catheter tube assembly may facilitate inserting the expandable body assembly into the urinary bladder and may extend through the urethra when the expandable body assembly is arranged in the urinary bladder.

In the expanded state, the expandable body assembly has a displacement volume V of more than 0.5 liters to allow for a substantial reduction of the intrapelvic volume. The width w2 of the center portion is smaller than 50% of the width w₃. The length l₁ is smaller than 25% of the total length l. With this shape, the expandable body assembly may for example be arranged in the urinary bladder such that the proximal portion is adjacent to the internal urethral orifice and the center portion is adjacent to the ureter orifices. The reduced width of the center portion allows for preventing the expandable body assembly from coming in contact with the ureter orifices, thereby allowing urine to flow into the urinary bladder. This may allow for leaving the expandable body assembly inside the urinary bladder for an extended amount of time, e.g. for more than 24 hours or for more than 48 hours. The widths w₁ and w₃ in the expanded state are larger than the corresponding width *w̃*₁ and *w̃*₃, respectively, in the collapsed state, i.e. both the proximal portion and the distal portion expand when expanding the at least one expandable body. Increasing the width of the proximal portion may e.g. be advantageous to prevent the expandable body assembly from accidentally being retracted into the urethra after expanding the at least one expandable body. In a preferred embodiment, the displacement volume V of the expandable body assembly in the expanded state is larger than 0.8 liters, preferably larger than 1.2 liters. In one example, the displacement volume of the expandable body assembly in the expanded state may for example be 1.5 liters. As detailed below, the catheter device may in some examples be configured to adjust a volume of the expandable body assembly in the expanded state. Alternatively or additionally, the width w₂ may be smaller than 20% of the width w₃, e.g. in order to reduce the probability that the expandable body assembly blocks the ureter orifices. Preferably, the width w₁ is at least two times as large as the width *w̃*₁. The width w₃ may also be at least two times as large as the width *w̃*₃, in some examples at least four times as larger as the width *w̃*₃. Furthermore, the length lᵢ may be smaller than 15% of the total length l, e.g. to more reliably ensure that the center portion is located adjacent to the ureter orifices when the proximal portion is adjacent to the internal urethral orifice.

The width w₂ may be smaller than 50% of the width w₁, preferably smaller than 30% of the width w₁ such that the expandable body assembly has a dumbbell-like or hourglass-like shape in the expanded state. This may further reduce the probability of the center portion coming in contact with the ureter orifices.

To facilitate arranging the expandable body assembly within the urinary bladder, the catheter device may be configured to adjust at least one parameter selected from a group consisting of the widths w₁, w₂, w₃ and w, the lengths l₁, l₂, l₃ and l and the displacement volume V. In one example, the catheter device is configured to adjust the total length l such that, when the expandable body assembly is arranged in the urinary bladder in the expanded state, the proximal portion is in contact with a bottom portion of the wall of the urinary bladder and the distal portion is in contact with an upper portion of the wall of the urinary bladder. This may be advantageous to prevent the expandable body assembly from moving within the urinary bladder. The catheter device may for example be configured to adjust the total length l by changing at least one of the lengths l₁, l₂, and l₃.

The catheter device may also be configured to adjust the width w₃ such that, when the expandable body assembly is arranged in the urinary bladder in the expanded state, the distal portion is in contact with a lateral portion of the wall of the urinary bladder. Preferably, the catheter device is configured to adjust the total length l, the width w₁ and/or the width w₃ such that, when the expandable body assembly is arranged in the urinary bladder in the expanded state, the expandable body assembly is in contact with at least 50%, preferably with at least 75% of a surface area of the wall of the urinary bladder.

In a preferred embodiment, the width w₂ is smaller than 2 cm, preferably smaller than 1 cm and/or the length l₂ is between 10% and 30% of the total length l. In one example, the width of the center portion may be uniform over the entire center portion or may deviate by less than 30%, preferably by less than 15% from the width w₂. The total length l may for example be between 10 cm and 20 cm, preferably between 12.5 cm and 17.5 cm. In some examples, the catheter device may be configured to adjust the total length l over a range between 12.5 cm and 17.5 cm, preferably between 10 cm and 20 cm. The width w₃ may be larger than the width w₁, preferably at least twice as large as the width w₁, e.g. to adapt a shape of the expandable body assembly to the shape of the urinary bladder. The width w₁ may for example be between 2 cm and 8 cm and the width w₃ may for example be larger than 10 cm, e.g. between 10 cm and 20 cm.

An outer surface of the proximal portion and/or an outer surface of the distal portion may have positive principal curvatures over at least 50%, preferably over at least 75% of the surface area of the respective portion in the expanded state, wherein the principal curvatures at a given point on a surface are the minimum and maximum values of the curvature of the surface at this point. Accordingly, positive principal curvatures correspond to a convex surface and negative principal curvatures correspond to a concave surface. In one example, the outer surface of the proximal portion and/or the outer surface of the distal portion maybe an ellipsoid. In other examples, the outer surface of the proximal portion may be a cylinder or a partial cylinder as detailed below. Additionally or alternatively, an outer surface of the center portion may have at least one principle curvature equal to or smaller than zero over at least 50%, preferably over at least 75% of the surface area of the center portion in the expanded state. In one example, the outer surface of the center portion may be a cylinder or a partial cylinder. In another example, the outer surface of the center portion may have a negative curvature in the direction of the longitudinal axis.

In some examples, the outer surface of the proximal portion and/or the outer surface of the center portion may comprise a recess or cut-out, e.g. for arranging an opening of a urine extraction tube as described below. The proximal portion and/or the center portion may for example be a partial cylinder with a recess or cut-out, e.g. a cylinder whose radial cross-section is a circular sector. The circular sector may for example have a central angle between 210° and 330°, in one example a central angle of 270°.

In some embodiments, the expandable body assembly is rotationally symmetric around the longitudinal axis extending from the proximal portion to the distal portion. The expandable body assembly may e.g. exhibit a discrete rotational symmetry, i.e. may be symmetric with respect to a rotation around the longitudinal axis by 360°/n with integer n > 1. The expandable body assembly may in particular exhibit a continuous rotational symmetry around the longitudinal axis, i.e. maybe symmetric with respect to a rotation around the longitudinal axis by any angle. This may facilitate arranging the expandable body assembly in the urinary bladder.

Preferably, the catheter device further comprises an urine extraction tube, wherein the urine extraction tube has at least one distal opening arranged in the center portion of the expandable body assembly. The urine extraction tube may e.g. be part of a catheter tube assembly attached to or connected to the proximal portion. The at least one distal opening of the urine extraction tube may be arranged such that the urine extraction tube provides a connection to the interior of the urinary bladder for fluids, e.g. to drain urine from the urinary bladder via the urine extraction tube. The at least one distal opening may e.g. be arranged on the outer surface of the center portion. In some examples, the at least one distal opening of the urine extraction tube may be located within a recess in the outer surface of the center portion, e.g. a recess formed by a partial cylinder. In one example, the urine extraction tube may have a plurality of distal openings distributed over the center portion.

The expandable body assembly may comprise an expandable body extending from the proximal portion to the distal portion, e.g. to adjust the widths w₁, w₂, and w₃ simultaneously by expanding the expandable body. In one example, the expandable body assembly comprises a single expandable body, e.g. a single expandable body extending from the proximal portion to the distal portion. In other examples, the expandable body assembly comprises a plurality of expandable bodies, e.g. a distal expandable body arranged in the distal portion of the expandable body assembly and a proximal expandable body arranged in the proximal portion of the expandable body assembly.

In a preferred embodiment, the at least one expandable body comprises an inflatable balloon comprising a flexible material enclosing an inner volume that is configured to receive an inflation medium. The inflatable balloon may for example comprise latex, polyurethane, silicone and/or a combination thereof. An inner surface and/or an outer surface of the inflatable balloon may be coated, wherein the coating may e.g. include a silicone elastomer, a hydrogel, polytetrafluoroethylene or a combination thereof.

Additionally or alternatively, the at least one expandable body may comprise other expandable elements, e.g. an element comprising a mechanically extendable element like a spring. In one example, the catheter device may comprise an insertion tube that is configured to be arranged in the urethra, wherein the expandable body assembly may be configured to be inserted into the urinary bladder through said insertion tube. The insertion tube may be configured to compress the mechanically extendable elements and the mechanically extendable elements may extend upon leaving the insertion tube.

The catheter device may further comprise an inflation tube, wherein the inflatable balloon is configured to receive the inflation medium through the inflation tube. The inflation tube may for example be part of a catheter tube assembly. The inflation tube may e.g. have a distal opening in communication with the inner volume of the inflatable balloon to supply the inflation medium to the inner volume. The opening may for example be arranged in a wall of the inflatable balloon or within its inner volume.

The center portion of the expandable body assembly may comprise at least one constraining element configured to limit a width of the center portion. In one example, the inflatable balloon may extend from the proximal portion to the distal portion. The constraining element may be configured to limit an expansion of the inflatable balloon in the center portion. The constraining element may for example comprise or consist of a material with smaller elasticity than a material of the inflatable balloon. The constraining element may for example be a ring or cylinder surrounding the inflatable balloon in the center portion. In other examples, the constraining element may be integrated into the inflatable balloon, e.g. a portion of the inflatable balloon with a larger wall thickness and/or comprising a different material than other portions of the inflatable balloon.

In a preferred embodiment, the inflation medium is a fluid, in particular an incompressible fluid like a liquid. The inflation medium may for example be water or a physiological salt solution. This may e.g. be advantageous in case of a leak in the inflatable balloon. Furthermore, it may facilitate adjusting a volume of the inflatable balloon by controlling the amount of liquid supplied to inflatable balloon as detailed below. In other examples, the inflation medium may be a gas or may be supplied to the inflatable balloon in a liquid phase and a phase transition to a gas phase may be induced to expand the expandable body assembly.

The catheter device may also comprise a volume control unit configured to adjust the volume of the inflatable balloon by adjusting a pressure in the inner volume of the inflatable balloon and/or by adjusting an amount of inflation medium supplied to the inner volume of the inflatable balloon. In other examples, the catheter device may comprise a connector to connect the catheter device to a volume control unit configured to adjust the volume of the inflatable balloon. The volume control unit may be configured to inject and/or extract inflation medium into/from the inner volume, e.g. by controlling a supply unit supplying the inflation medium. The volume control unit may be configured to control the amount of inflation medium supplied to the inner volume by injecting and/or extracting inflation medium, e.g. to supply a predefined volume of an incompressible inflation medium such as a liquid. Alternatively or additionally, the volume control unit may be configured to control the pressure in the inner volume of the inflatable balloon by injecting and/or extracting inflation medium, e.g. to reach a predefined pressure in the inner volume. The volume control unit may be configured to adjust the volume of the inflatable balloon in the expanded state at least within a range between 1 liter and 1.5 liters, preferably at least within a range between 0.5 liters and 2 liters, most preferably at least within a range between 0.5 liters and 3 liters.

The catheter device may further comprise a pressure sensor configured to determine a pressure in the inner volume of the inflatable balloon and/or a pressure on an outer surface of the expandable body assembly. The pressure sensor may for example be a piezoresistive, piezoelectric, inductive or capacitive pressure sensor. The pressure sensor may e.g. be arranged in the inner volume, in or adjacent to the wall of the inflatable balloon or on an outer surface of the expandable body assembly. In other examples, the pressure sensor may be integrated into the inflation tube or another element of the catheter device exposed to the inflation medium, e.g. a pump or a tube in the supply unit.

As mentioned above, the expandable body assembly may comprise more than one expandable body. The expandable body assembly may for example comprise a distal expandable body arranged in the distal portion of the expandable body assembly and a proximal expandable body arranged in the proximal portion of the expandable body assembly. The catheter device may be configured such that the expandable bodies may be expanded independently of one another, e.g. to expand the proximal expandable body prior to expanding the distal expandable body or to expand the proximal and distal expandable bodies to different volumes and/or pressures. In some examples, the proximal expandable body may extend from the proximal portion to the center portion. A distal end of the proximal inflatable body may e.g. be directly adjacent to a proximal end of the distal inflatable body. The proximal expandable body may for example be an ellipsoid, a cylinder or a partial cylinder. The expandable body assembly may also comprise a central expandable body arranged in the center portion.

The proximal expandable body and the distal expandable body may each comprise an inflatable balloon configured to receive an inflation medium. The catheter device may further comprise a proximal inflation tube and a distal inflation tube, wherein the inflatable balloon in the proximal portion is configured to receive an inflation medium through the proximal inflation tube and the inflatable balloon in the distal portion is configured to receive an inflation medium through the distal inflation tube. The catheter device may be configured such that there is no connection between the proximal inflation tube and the distal inflation tube to exchange inflation medium. In other examples, the catheter device may comprise a single inflation tube to supply inflation medium to both the inflatable balloon in the proximal portion and the inflatable balloon in the distal portion.

In a preferred embodiment, the catheter device comprises an insertion tube extending through the proximal portion and the center portion of the expandable body assembly. The distal portion of the expandable body assembly may be configured to be inserted into the urinary bladder through the insertion tube in the collapsed state. The distal portion may in particular be part of an independent catheter assembly. The insertion tube may e.g. be part of the catheter tube assembly, which may also comprise the urine extraction tube and/or the proximal inflation tube. In this way, the proximal portion and the center portion of the expandable body assembly may first be inserted into the urinary bladder via the urethra, e.g. to provide access to the urinary bladder via the urine extraction tube similar to a conventional urinary catheter. If needed, the distal portion may subsequently be inserted through the insertion tube, e.g. using the distal inflation tube to thread the distal portion through the insertion tube. This may be advantageous to increase the flexibility when employing the catheter device. The distal portion may in particular be inserted after expanding the proximal portion and/or the center portion to the expanded state, while the distal portion is in the collapsed state.

### LIST OF FIGURES

In the following, a detailed description of the invention and exemplary embodiments thereof is given with reference to the figures. The figures show schematic illustrations of
Fig. 1a: a transurethral catheter device in an expanded state according to an exemplary embodiment of the invention;
Fig. 1b: the transurethral catheter device of Fig. 1a in a collapsed state;
Fig. 2: a transurethral catheter device with an inflatable balloon and a constraining element in accordance with an embodiment of the invention;
Fig. 3: a transurethral catheter device with two expandable bodies according to an exemplary embodiment of the invention;
Fig. 4: a transurethral catheter device with a supply unit in accordance with an embodiment of the invention;
Fig. 5: a flow diagram illustrating a method for using a transurethral catheter device according to an example not claimed in the appended claims;
Fig. 6a: a transurethral catheter device with two expandable bodies and an insertion tube in a partially expanded state in accordance with an embodiment of the invention;
Fig. 6b: a cross section of the center portion of the catheter device of Fig. 6a in an expanded state;
Fig. 6c: the transurethral catheter device of Fig. 6a in the expanded state; and
Fig. 7: a flow diagram illustrating a method for using a transurethral catheter device with two independent catheter assemblies according to an example not claimed in the appended claims.

### DESCRIPTION OF THE PREFERRED EMBODIMENTS

The invention is defined in claim 1. Preferable embodiments are defined in the dependent claims.

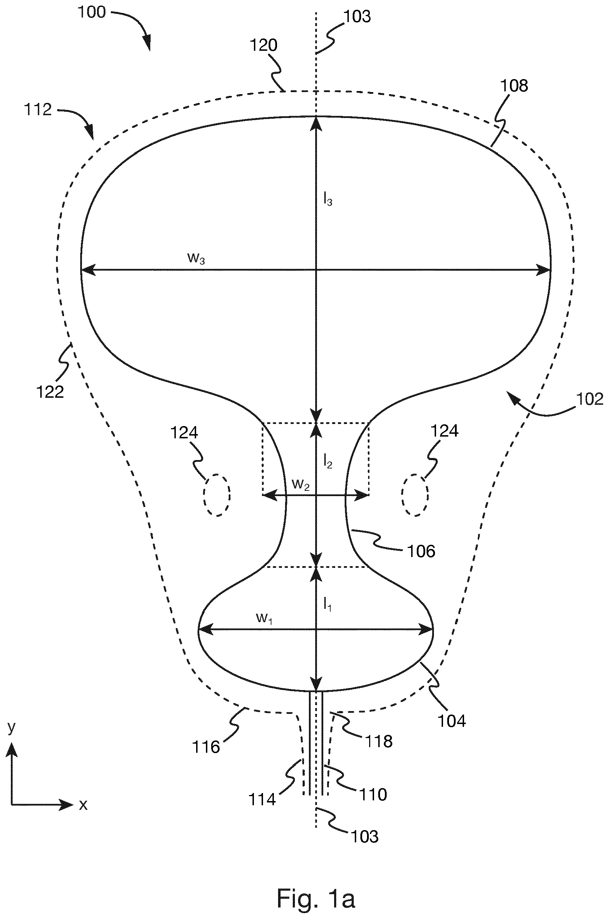

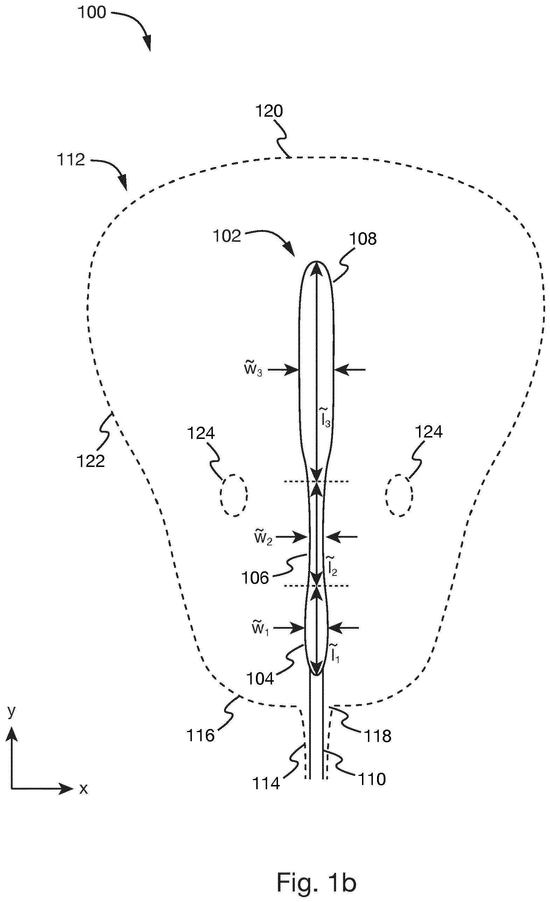

Figs. 1a and 1b depict a schematic illustration (not to scale) of a sectional view of a transurethral catheter device 100 in accordance with an embodiment of the invention. The catheter device 100 comprises an expandable body assembly 102 with at least one expandable body. By expanding the at least one expandable body, the expandable body assembly 102 can be expanded from a collapsed state to an expanded state. The catheter device 100 is shown with the expandable body assembly 102 in the expanded state in Fig. 1a and with the expandable body assembly 102 in the collapsed state in Fig. 1b.

The expandable body assembly 102 has a proximal portion 104, a center portion 106 and a distal portion 108. In the expanded state, the portions 104, 106, and 108 are arranged along a longitudinal axis 103 of the expandable body assembly 102 such that the proximal portion 104 is directly adjacent to the center portion 106 and the center portion 106 is directly adjacent to the distal portion 108. The longitudinal axis 103 may e.g. be defined as the axis around which the expandable body assembly has the smallest moment of inertia in the expanded state and is aligned with the y-axis in the example shown in Fig. 1a. The portions 104, 106, and 108 differ in their physical dimensions as detailed below. In some examples, the portions 104, 106, and 108 may have an identical internal structure, e.g. comprise or consist of the same materials. The portions 104, 106, and 108 may in particular be virtual axial segments of the expandable body assembly 102 along the longitudinal axis 103 as in the example shown in Figs. 1a and 1b, in which the virtual borders between the portions 104, 106, and 108 are indicated by horizontal dotted lines. In other examples, the portions 104, 106 and 108 may have different internal structures, e.g. comprise or consist of different materials.

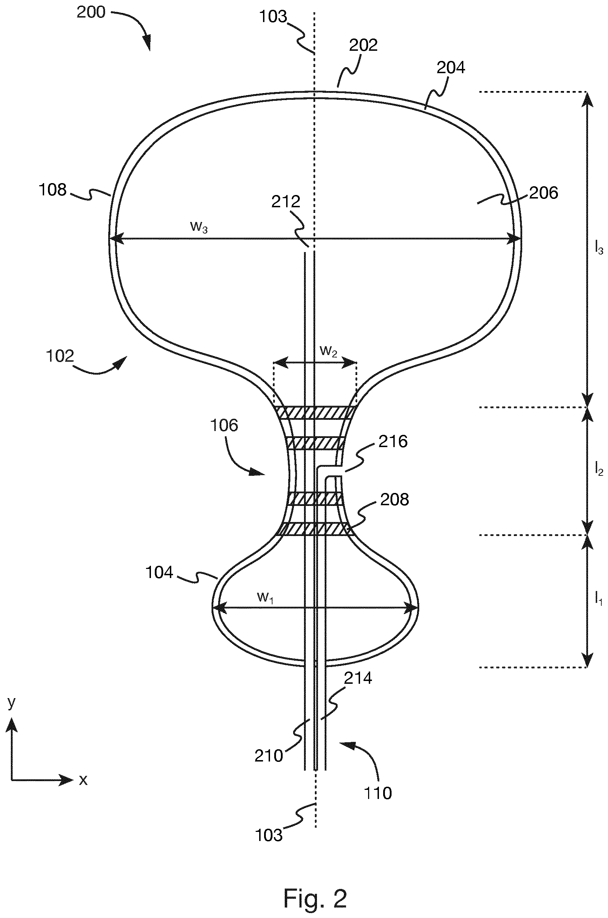

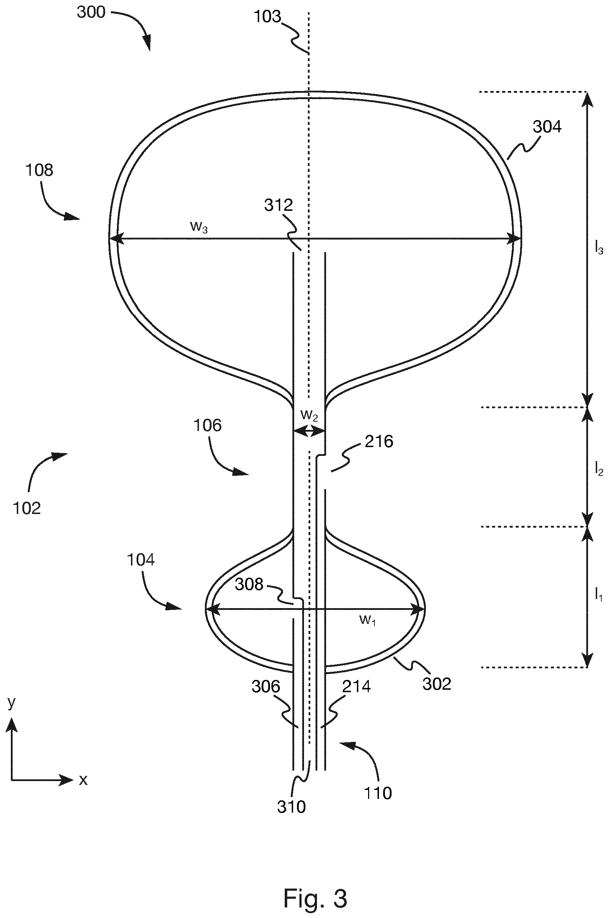

The at least one expandable body may for example comprise one or more expandable elements, e.g. an inflatable balloon as described below with reference to Figs. 2 and 3. Additionally or alternatively, the at least one expandable body may comprise other expandable elements, e.g. an element comprising a mechanically extendable element like a spring as detailed below.

The at least one expandable body may be arranged in the proximal portion 104, the center portion 106 and/or the distal portion 108. In one example, the expandable body assembly 102 may comprise an expandable body that extends from the proximal portion 104 through the center portion 106 to the distal portion 108. The expandable body assembly 102 may comprise a plurality of expandable bodies, each being configured to be expanded, e.g. one expandable body arranged in the distal portion 108 and another expandable body arranged in the proximal portion 104. In some examples, the catheter device 100 may be configured such that each of the expandable bodies may be expanded independently of the other expandable bodies.

The catheter device 100 further comprises a catheter tube assembly 110 that is attached to the proximal portion 104. In one example, the catheter tube assembly 110 may be formed integrally with the expandable body assembly 102. In another example, the catheter tube assembly 110 may be detachably connected to the expandable body assembly 102 through a connector. The catheter tube assembly 110 may for example be similar to the catheter tube assembly of the catheter device 200 of Fig. 2 or the catheter device 300 of Fig. 3 described below. Preferably, the catheter tube assembly 110 comprises an urine extraction tube that is in communication with the inner volume of the urinary bladder 112, e.g. similar to the urine extraction tube 214 of the catheter device 200. In the example shown in Figs. 1a and 1b, the expandable body assembly 102 has been inserted into the urinary bladder 112 of a patient via the urethra 114. Correspondingly, the catheter tube assembly 110 may extend through the urethra 114 and may provide a connection to the expandable body assembly 102 and/or to the urinary bladder 112 from the outside. The catheter tube assembly 110 may furthermore facilitate arranging the extendable body assembly 102 in the urinary bladder 112 as detailed below.

In one example, the catheter device 100 may comprise an insertion tube (not shown) that is configured to be arranged in the urethra 114, wherein the expandable body assembly 102 or a part thereof may be configured to be inserted into the urinary bladder 112 through said insertion tube. The insertion tube may e.g. be configured to compress a mechanically extendable element of the expandable body assembly 102 when the expandable body assembly 102 is inside the insertion tube. When the expandable body assembly 102 leaves the insertion tube and enters the urinary bladder 112, the mechanically extendable element may extend, thereby expanding the expandable body assembly 102. In one example, a mechanically extendable element may be arranged in the proximal portion 104, wherein the mechanically extendable element extends when entering the urinary bladder 112, thereby increasing the width of the proximal portion 104. This may e.g. be advantageous to prevent the proximal portion 104 from accidentally being retracted into the urethra 114 again as a force exceeding a certain threshold may be required to compress the mechanically extendable element in order to pull the proximal portion 104 into the urethra 114.

The proximal portion 104 has a length l₁ and a width w₁ in the expanded state and a length *l̃*₁ and a width *w̃*₁ in the collapsed state. The center portion 106 has a length l₂ and a width w₂ in the expanded state and a length *l̃*₂ and a width *w̃*₂ in the collapsed state. The distal portion 108 has a length l₃ and a width w₃ in the expanded state and a length *l̃*₃ and a width *w̃*₃ in the collapsed state. The length of a portion is defined as the largest extent of the respective portion in the direction of the longitudinal axis 103. The width of a portion is defined as the largest radially averaged width of the respective portion perpendicular to the longitudinal axis 103, wherein radial averaging of the width corresponds to taking the average of the width in a plane perpendicular to the longitudinal axis 103. In some examples, the expandable body assembly 102 may be rotationally symmetric around the longitudinal axis 103 and may e.g. exhibit a continuous rotational symmetry such that the width of the expandable body assembly 102 is the same in every direction perpendicular to the longitudinal axis 103 at a given point along the longitudinal axis 103. In other examples, at least one of the portions 104, 106, and 108 may be rotationally symmetric with respect to the longitudinal axis 103.

In the example shown in Figs. 1a and 1b, a total length l of the expandable body assembly 102 in the expanded state is given by l = l₁ + l₂ + l₃ and a total length *l̃* of the expandable body assembly 102 in the collapsed state is given by *l̃* = *l̃*₁, + *l̃*₂ + *l̃*₃, wherein the total length of the expandable body assembly 102 is the largest extent of the expandable body assembly 102 in the direction of the longitudinal axis 103. The total widths w and *w̃* of the expandable body assembly are the largest width among the widths of the individual portions, i.e. equal to w₃ and *w̃*₃, respectively, in the example shown in Figs 1a and 1b.

The lengths and widths in the expanded state are larger than the respective quantities in the collapsed state such that a displacement volume V of the expandable body assembly 102 in the expanded state is larger than a displacement volume *Ṽ* of the expandable body assembly 102 in the collapsed state. Thereby, a volume of the urinary bladder 112 may be increased by expanding the expandable body assembly 102 from the collapsed state to the expanded state. Furthermore, the dimensions of the expandable body assembly 102 in the collapsed state are sufficiently small in order to insert the expandable body assembly 102 into the urinary bladder 112 through the urethra 114. The displacement volume in the collapsed state *Ṽ* may therefore be much smaller than the displacement volume V, e.g. less than 5%, preferably less than 1% of V. In one example, the total width *w̃* of the expandable body in the collapsed state may be less than 10 mm, preferably less than 5 mm.

The displacement volume V may for example be in the range between 0.5 liters and 3 liters, e.g. 1.5 liters. The width w₂ is smaller than 50%, preferably smaller than 20% of the width w₃. As shown in Fig. 1a, the width w₂ may also be smaller than 50%, preferably smaller than 30% of the width w₁. In one example, the width w₁ may be 5 cm, the width w₂ may be 1.5 cm and the width w₃ may be 15 cm. The length l₁ is smaller than 25%, preferably smaller than 15% of the total length l. The total length l may for example be 15 cm and the length l₁ may e.g. be 2 cm. The length l₂ may be between 10% and 30% of the total length l, e.g. 3 cm. Correspondingly, the length l₃ may for example be 10 cm. The displacement volume of the distal portion 108 may be more than 75%, in some examples more than 90% of the displacement volume V.

In the example shown in Fig. 1a, the expandable body assembly 102 is arranged such that the proximal portion 104 is adjacent to a bottom portion 116 of the wall of the urinary bladder 112 that is in the vicinity of the internal urethral orifice 118, and the distal portion 108 is adjacent to an upper portion 120 and a lateral portion 122 of the wall of the urinary bladder 112. In some cases, the proximal portion 104 may even be in contact with the bottom portion 116 and the distal portion 108 may be in contact with the upper portion 120 and/or the lateral portion 122. The catheter tube assembly 110 may facilitate arranging the expandable body assembly 102 in the urinary bladder 112 into the configuration shown in Fig. 1a since the catheter tube assembly 110 is connected to the proximal portion 104 and thus may ensure that the proximal portion 104 is located adjacent to the internal urethral orifice 118.

The shape of the expandable body assembly 102 in the expanded state is such that the center portion 106 is located adjacent to the ureter orifices 124 of the urinary bladder 112 when the expandable body assembly 102 is arranged in the urinary bladder 112 as shown in Fig. 1a. Due to the smaller width w₂ of the center portion 106, which is preferably smaller than the typical distance between the ureter orifices 124, the center portion 106 is not in contact with the ureter orifices 124. In this way, an uninterrupted flow of urine through the ureters into the urinary bladder 112 may be ensured. A rotationally symmetric shape of the center portion 106 and/or of the expandable body assembly 102 around the longitudinal axis 103 may facilitate arranging the expandable body assembly 102 in the urinary bladder 112 such that the ureter orifices 124 are not blocked by the expandable body assembly 102 irrespective of the orientation of the expandable body assembly 102 around the longitudinal axis 103.

The catheter device 100 may be configured to adjust at least one of the parameters comprising the widths w₁, w₂, w₃ and w, the lengths l₁, l₂, l₃ and l and the displacement volume V, e.g. in order to facilitate arranging the expandable body assembly 102 within the urinary bladder 112. The catheter device 100 may for example be configured to adjust the at least one parameter such that the proximal portion 104 is in contact with the bottom portion 116 of the wall of the urinary bladder 112 and/or such that the distal portion 108 is in contact with the upper portion 120 and/or the lateral portion 122 of the wall of the urinary bladder 112, e.g. by changing the lengths l₁ and l₃ and/or the width w₃. In one example, the catheter device 100 may be configured to adjust the at least one parameter such that expandable body assembly 102 is in contact with at least 50%, preferably with at least 75% of the surface area of the wall of the urinary bladder 112.

The shape of the expandable body assembly 102 may be adapted to the shape of the urinary bladder 112 of a human, e.g. in order to reduce the mechanical stress on the urinary bladder 112 when expanding the expandable body assembly 102 and/or to ensure that the expandable body assembly 102 is securely fitted in the urinary bladder 112. An outer surface of the proximal portion 104 and/or an outer surface of the distal portion 108 may for example have positive principle curvatures over at least 50%, preferably over at least 75% of the surface area of the respective portion in the expanded state, e.g. as in the example shown in Fig. 1a. In other examples, the proximal portion 104 may have a cylindrical shape, e.g. a cylinder with an axis parallel to the longitudinal axis 103, or may be a partial cylinder. Preferably, the proximal portion 104, the distal portion 108, and/or the expandable body assembly 102 comprises or consists of a flexible material, e.g. such that the shape of the expandable body assembly 102 adapts to the shape of the urinary bladder 112 when expanding the expandable body assembly 102.

In the expanded state, an outer surface of the center portion 106 may have at least one principle curvature equal to or smaller than zero over at least 50%, preferably over at least 75% of the surface area of the center portion 106. In the example shown in Fig. 1a, the center portion 106 has a negative principle curvature, i.e. a concave shape, along the longitudinal axis 103, which may e.g. be advantageous to increase the distance to the ureter orifices 124. In other examples, the center portion 106 may be a cylinder, i.e. exhibit a principle curvature of zero along the longitudinal axis 103.

Fig. 2 shows a schematic illustration (not to scale) of a sectional view of a transurethral catheter device 200 according to an exemplary embodiment of the invention. Similar to the catheter device 100, the catheter device 200 comprises an expandable body assembly 102 having a proximal portion 104, a center portion 106 and a distal portion 108 and a catheter tube assembly 110. In Fig. 2, the catheter device 200 is shown with the expandable body assembly 102 in the expanded state.

In the example shown in Fig. 2, the expandable body assembly 102 comprises an inflatable balloon 202 as an expandable body and the portions 104, 106, and 108 are virtual axial segments of the inflatable balloon 202 that differ in their physical dimensions as detailed above with reference to Fig. 1a. The inflatable balloon 202 comprises a wall 204 that consists of a flexible material and encloses an inner volume 206. The wall 204 may for example be made of latex, polyurethane, silicone and/or a combination thereof. An inner surface and/or an outer surface of the wall 204 may be coated, e.g. to reduce friction or to decrease the hydrophobicity of the respective surface. The coating may for example include a silicone elastomer, a hydrogel, polytetrafluoroethylene or a combination thereof.

The inner volume 206 is configured to receive an inflation medium, wherein a volume of the inflatable balloon 202 may e.g. be adjusted by adding inflation medium to the inner volume 206 or extracting inflation medium from the inner volume 206. The flexible material of the wall 204 may allow for changing V, l₁, l₂, l₃, l, w₁, w₂, w₃ and/or w in the expanded state by adjusting the amount of inflation medium supplied to the inner volume 206. Preferably, the inflation medium is a fluid, in particular an incompressible fluid like a liquid, for example a physiological salt solution. In other examples, the inflation medium may be a gas. In one example, the inflation medium may be supplied to the inner volume 206 in a liquid phase and a phase transition to a gas phase may be induced to expand the expandable body assembly, e.g. through a chemical reaction and/or a change in temperature.

The inflatable balloon 202 may be configured to be expanded up to a volume of at least 150%, preferably of at least 200% of the displacement volume V by supplying inflation medium to the inner volume 206 without creating leaks in the wall 204 or otherwise damaging the inflatable balloon 202, e.g. by choosing an appropriate thickness and/or material composition of the wall 204. This may provide a safety margin when expanding the expandable body assembly 102 to the expanded state. The inflatable balloon 202 may e.g. be configured to be expanded up to a volume of at least 2.5 liters without creating leaks in the wall 204 or otherwise damaging the inflatable balloon 202. A minimum thickness of the wall 204 in the expanded state may for example be larger than 0.5 mm, preferably larger than 1 mm.

The shape of the inflatable balloon 202 in the expanded state may for example be determined by a surface area of the wall 204, a thickness of the wall 204 and/or a material composition of the wall 204 in the collapsed state in different parts of the inflatable balloon 202. In the example shown in Fig. 2, the inflatable balloon 202 extends from the proximal portion 104 through the center portion 106 to the distal portion 108. In one example, the thickness of the wall 204 may be larger in the center portion 106 than in the proximal portion 104 and/or in the distal portion 108 in order to achieve a smaller width w₂ in the center portion 106 than in the proximal portion 104 and in the distal portion 108. Alternatively or additionally, the surface area of the wall 204 may be larger in the distal portion 108 than in the center portion 106 and/or in the proximal portion 104. As the wall 204 consists of a flexible material, the shape of the inflatable balloon 202 may adapt to the shape of the urinary bladder 112, e.g. when the wall 204 comes in contact with the wall of the urinary bladder 112.

The expandable body assembly 102 of the catheter device 200 further comprises constraining elements 208 that are configured to limit the width w₂ of the center portion 106. In this example, the constraining elements 208 are ring-shaped reinforcements of the center portion 106. The constraining elements 208 may be integral parts of the center portion 106, e.g. regions with a thicker wall 204 and/or with a different material composition. The constraining elements 208 may for example comprise or consist of a material with a smaller elasticity than other parts of the center portion 106. In other examples, the constraining elements 208 may be separate from the center portion 106 and may for example be ring-shaped elements that are attached to the center portion 106 and surround the center portion 106.

The catheter device 200 may further comprise a removable cover (not shown), which may cover at least a part of the expandable body assembly 102, e.g. an outer surface of the distal portion 108. The cover may for example be removed after inserting the expandable body assembly 102 into the urinary bladder 112 and prior to expanding the expandable body assembly 102. A surface of the cover may be coated, wherein the coating may e.g. include a silicone elastomer, a hydrogel, polytetrafluoroethylene or a combination thereof.

The catheter tube assembly 110 comprises an inflation tube 210 with a distal opening 212 that is in communication with the inner volume 206, i.e. opens towards the inner volume 206. In the example shown in Fig. 2, the distal opening 212 is arranged inside the inner volume 206 of the inflatable balloon 202. Thereby, inflation medium can be supplied to the inner volume 206 through the inflation tube 210. In other examples, the distal opening 212 may be arranged in the outer wall 204 of the inflatable balloon 202. In some examples, the inflation tube 210 may comprise a plurality of openings in communication with the inner volume 206 (not shown). The inflation tube 210 may e.g. comprise or consist of latex, polyurethane, silicone and/or a combination thereof. The material composition and/or the wall thickness of the inflation tube 210 may be chosen such that a diameter of the inflation tube 210 outside of the expandable body assembly 102 changes by less than 50%, preferably by less than 25% when expanding the inflatable balloon 202 from the collapsed state to the expanded state. When the inflatable balloon 202 is in the expanded state, a diameter of the inflation tube 210 outside of the expandable body assembly 102 may for example be less than 2 mm, preferably less than 1 mm. The inflation tube 210 may further comprises a valve, which may e.g. be opened to inject inflation medium into the inflatable balloon 202 and subsequently be closed to prevent the inflation medium from leaving the inflatable balloon 202.

The catheter tube assembly 110 further comprises an urine extraction tube 214 with a distal opening 216 that is arranged in the center portion 106 of the expandable body assembly 102. The distal opening 216 may for example be arranged on an outer surface of the wall 204 as shown in Fig. 2 such that the distal opening 216 is in communication with the inner volume of the urinary bladder 112 when the expandable body assembly 102 is arranged in the urinary bladder 112. In this way, the urine extraction tube 214 may be used to drain urine from the urinary bladder 112 as well as to inject a substance into the urinary bladder 112, e.g. for treatment or diagnostic purposes. In some examples, the urine extraction tube 214 may comprise a plurality of distal openings arranged in the center portion 106 (not shown). The urine extraction tube 214 may be made of the same material as the inflation tube 210 and may have the same diameter. In one example, the urine extraction tube 214 may enclose at least a part of the inflation tube 210 or vice versa, i.e. the inflation tube 210 may e.g. be arranged within the urine extraction tube 214.

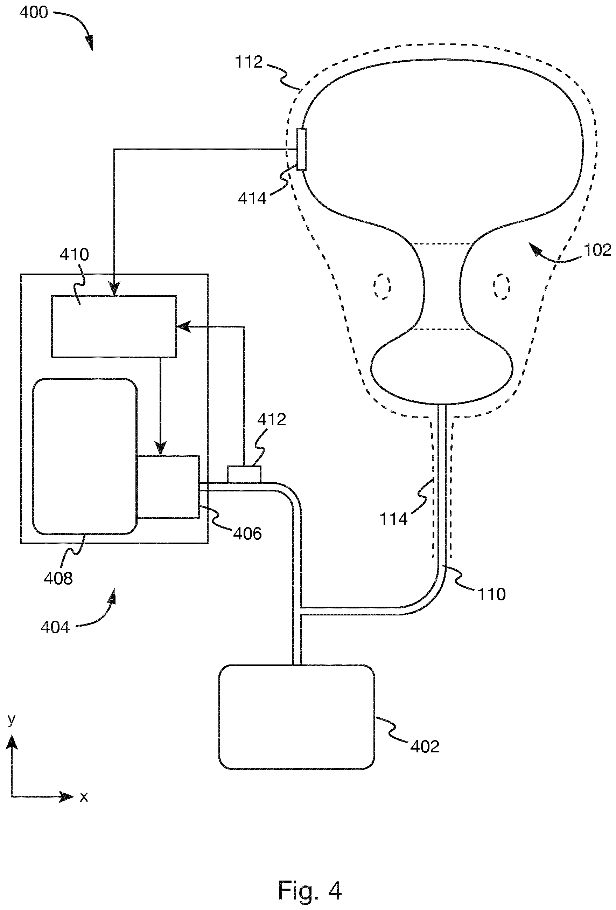

In the example shown in Fig. 2, the catheter tube assembly 110 is attached to the proximal portion 104 and extends outwards from the proximal portion 104. The catheter tube assembly 110 may for example have a length between 0.5 m and 2 m such that the catheter tube can extend through the urethra 114 and can be connected to an external supply unit, e.g. as detailed below with reference to Fig. 4. In other examples, the catheter tube assembly 110 may terminate at a connector arranged on or adjacent to an outer wall of the expandable body assembly 102, e.g. in the proximal portion 104, wherein the connector is configured to receive an external catheter tube assembly and to connect the external catheter tube assembly to the catheter tube assembly 110.

Fig. 3 depicts a schematic illustration (not to scale) of a sectional view of a transurethral catheter device 300 in accordance with an embodiment of the invention. Similar to the catheter devices 100 and 200, the catheter device 300 comprises an expandable body assembly 102 having a proximal portion 104, a center portion 106 and a distal portion 108 and a catheter tube assembly 110. In Fig. 3, the catheter device 300 is shown with the expandable body assembly 102 in the expanded state.

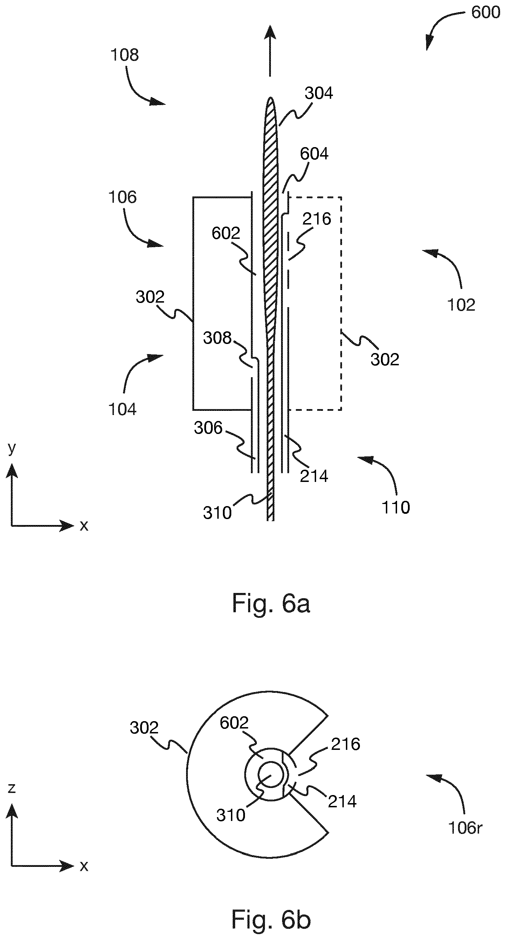

The expandable body assembly 102 of the catheter device 300 comprises two expandable bodies, namely a proximal inflatable balloon 302 and a distal inflatable balloon 304. The proximal inflatable balloon 302 is arranged in the proximal portion 104 of the expandable body assembly 102 and the distal inflatable balloon 304 is arranged in the distal portion 108 of the expandable body assembly 102. The center portion 106 of the expandable body assembly 102 on the other hand does not comprise an expandable body, but only comprises a part of the catheter tube assembly 110. Correspondingly, the width w₂ of the center portion 106 in the expanded state may be comparable to the width *w̃*₂ of the center portion 106 in the collapsed state, e.g. less than 150% of *w̃*₂. In other embodiments, the proximal inflatable balloon 302 may extend from the proximal portion 104 to the center portion 106 (not shown), e.g. similar to the embodiment described below with reference to Figs. 6a-6c. A distal end of the proximal inflatable balloon 302 may e.g. be directly adjacent to a proximal end of the distal inflatable balloon 304.

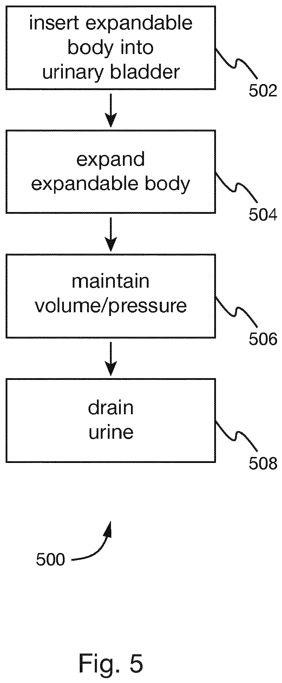

Similar to the inflatable balloon 202 of the catheter device 200, each of the proximal and distal inflatable balloons 302 and 304 comprises an outer wall surrounding an inner volume that is configured to receive an inflation medium. To supply the inflation medium, the catheter tube assembly 110 comprises a proximal inflation tube 306 and a distal inflation tube 310 in addition to an urine extraction tube 214. The proximal inflation tube 306 has a distal opening 308 that is in communication with the inner volume of the proximal inflatable balloon 302. The distal inflation tube 310 has a distal opening 312 that is in communication with the inner volume of the distal inflatable balloon 304. In this way, the inflation medium may be supplied to the balloons 302 and 304 independently, e.g. to expand the balloons 302 and 304 to different volumes and/or pressures or to use different inflation media for the balloons 302 and 304. In particular, the balloons 302 and 304 may be expanded at different points in time when expanding the expandable body assembly 102 to the expanded state, e.g. as detailed below with reference to Fig. 5.

The proximal inflatable balloon 302 and/or the distal inflatable balloon 304 may have an ellipsoidal shape, in particular a spherical shape in the expanded state. In other examples, the proximal inflatable balloon 302 may have a cylindrical shape or the shape of a partial cylinder, e.g. as detailed below with reference to Figs. 6a-6c. In some examples, the volume of the distal inflatable balloon 304 in the expanded state may be at least five times as large, in one example at least ten times as large as the volume of the proximal inflatable balloon 302 in the expanded state. The catheter device 300 may further comprise a removable cover (not shown), which may cover at least a part of the expandable body assembly 102, e.g. an outer surface of the distal inflatable balloon 304.

Fig. 4 schematically illustrates a transurethral catheter device 400 in accordance with an embodiment of the invention. The catheter device 400 comprises an expandable body assembly 102, which may for example be similar to the expandable body assembly of the catheter device 100, 200 or 300 described above. In Fig. 4, the expandable body assembly 102 is arranged in the urinary bladder 112 of a patient such that a catheter tube assembly 110 attached to or connected to the expandable body assembly 102 extends through the urethra 114 of the patient to the outside. The catheter tube assembly 110 may for example comprise an urine extraction tube similar to the urine extraction tube 214 shown in Figs. 2 and 3 and at least one inflation tube similar to the inflation tube 210 or 310 shown in Fig. 2 and 3, respectively, to supply an inflation medium to the expandable body assembly 102. The catheter tube assembly 110 is connected to an urine reservoir 402 that is configured to receive urine drained from the urinary bladder 112 via the catheter tube assembly 110, e.g. through the urine extraction tube 214.

The catheter tube assembly 110 is furthermore connected to a supply unit 404 that is configured to provide the inflation medium, e.g. through the at least one inflation tube 210. The supply unit 404 comprises a pump 406 that is connected with a supply reservoir 408. The supply reservoir 408 is configured to store a certain amount of inflation medium, for example 3 liters to 5 liters of inflation medium. In some examples, the supply reservoir 408 may be an infusion bag connected to the supply unit 404 and/or to the pump 406. The pump 406 may for example be configured to pump inflation medium from the supply reservoir 408 into the expandable body assembly 102. Additionally, the pump 406 may be configured to pump inflation medium from the expandable body assembly 102 into the supply reservoir 408 through the catheter tube assembly 110.

The supply unit 404 further comprises a control unit 410 that is configured to control operation of the pump 406. The supply unit 404 may for example be connected to a control panel of the supply unit 404, e.g. such that an operator of the supply unit 404 can pump inflation medium to/from the expandable body assembly 102 by pressing a button and/or control a set point value for the displacement volume V, a pressure and/or an amount of inflation medium to be supplied, e.g. by rotating a switch.

The catheter device 400 further comprises a flow meter 412 that is configured to measure an amount of the inflation medium transported through the catheter tube assembly 110, e.g. a volume of the transported inflation medium. The flow meter 412 may for example be a mechanical flow meter, a pressure-based flow meter, an optical flow meter or an electromagnetic flow meter. The flow meter 412 is coupled to the control unit 410. The control unit 410 may for example be configured to determine the amount of inflation medium supplied to the expandable body assembly 102 via the flow meter 412, e.g. by measuring the volume of the inflation medium injected into and/or extracted from the expandable body assembly 102. In other examples, the control unit 410 may determine the amount of inflation medium in the supply reservoir 408 to determine the amount of inflation medium supplied to the expandable body assembly 102. The control unit 410 may furthermore be configured to supply a predetermined amount of inflation medium to the expandable body assembly 102, wherein the predetermined amount may for example be set by an operator using the control panel.

The catheter device 400 also comprises a pressure sensor 414 that is arranged in the expandable body assembly 102. The pressure sensor 414 may for example be arranged on an outer surface of the expandable body assembly 102, e.g. an outer surface of the wall 204 of the inflatable balloon 202, and may be configured to measure a pressure in the vicinity of the expandable body assembly 102. In other examples, the pressure sensor 414 may be arranged on an inner surface of the wall 204 or within the inner volume 206 of the inflatable balloon 202, e.g. to measure the pressure in the inner volume 206. Alternatively, the pressure sensor 414 may be arranged in other elements of the catheter device 400 that are in contact with the inflation medium, e.g. in a sidewall of the at least one inflation tube 210 or within the supply unit 404. The control unit 410 is coupled to the pressure sensor 414 and may be configured to read-out the pressure measured by the pressure sensor 414. The control unit 410 may also be configured to control the pressure measured by the pressure sensor 414, e.g. to a predetermined set point specified by the operator, by injecting and/or extracting inflation medium into/from the expandable body assembly 102 by the pump 406.

Fig. 5 depicts a flowchart of a method 500 to treat a patient using a transurethral catheter device in accordance with an embodiment of the invention. The method 500 may for example be performed with any one of the catheter devices 100, 200, 300 and 400 and will be described the following with reference to Figs. 2 to 4.

In a first step 502, the expandable body assembly 102 is prepared in the collapsed state and inserted into the urinary bladder 112 of the patient via the urethra 114. The expandable body assembly 102 may for example be inserted into the urethra 114 by first inserting the distal portion 108 of the expandable body assembly 102 into the urethra 114, subsequently inserting the center portion 106 and the proximal portion 104 and finally pushing the expandable body assembly 102 through the urethra 114 into the urinary bladder 112 using the catheter tube assembly 110.

Once the expandable body assembly 102 has been fully inserted into the urinary bladder 112, the expandable body assembly 102 is expanded from the collapsed state to the expanded state by expanding the at least one expandable body in step 504. In the examples of the catheter devices 200 and 300, the expandable body assembly 102 may be expanded by injecting inflation medium into the inflatable balloons 202 and 302/304, respectively. In some examples, the expandable body assembly 102 may be expanded to a predefined displacement volume V and/or pressure, e.g. using the control unit 410. The displacement volume V and/or the pressure that the expandable body assembly 102 is expanded to may be chosen depending on a weight, size, gender and/or condition of the patient. For example, a larger displacement volume V may be used for a taller and heavier patient than for a smaller and lighter patient. Additionally or alternatively, a different catheter device may be used depending on the weight, size, gender and/or condition of the patient, e.g. a catheter device with a displacement volume V of 1.5 liters for a male patient and a catheter device with a displacement volume V of 1.2 liters for a female patient.

In one example, a catheter device is used that comprises a plurality of expandable bodies which can be expanded independently, e.g. the catheter device 300 with the distal inflatable balloon 304 and the proximal inflatable balloon 302. In this case, the expandable bodies may be expanded at different points in time. For example, after inserting the expandable body assembly 102 into the urinary bladder 112, the proximal inflatable balloon 302 may be expanded first by injecting inflation medium through the proximal inflation tube 306. This may prevent the expandable body assembly 102 from entering the urethra 114 again. Subsequently, the distal inflatable balloon 304 may be expanded by injecting inflation medium through the distal inflation tube 310. The distal inflatable balloon 304 may also be expanded to a different pressure than the proximal inflatable balloon 302, e.g. to a higher pressure to reach a larger displacement volume for the distal inflatable balloon 304.

Subsequently, in step 506, the displacement volume V and/or the pressure is maintained, e.g. by injecting and/or extracting inflation medium to/from the expandable body assembly 102 using the supply unit 404 and/or by the pump 406. The control unit 410 of the supply unit 404 may for example be configured to regulate the displacement volume V and/or the pressure to the predefined set point. In one example, the control unit 410 is configured to regulate the pressure to the predefined set point and may thereby react to a pressure change in the patient's abdomen, e.g. resulting from a release of urine.

While the expandable body assembly 102 is arranged in the urinary bladder 112, urine may be drained from the urinary bladder 112 via the urine extraction tube 214 in step 508. Urine may for example be drained from the urinary bladder 112 as a result of the increased pressure created by the expansion of the expandable body assembly 102. To account for this, the control unit 410 may be configured to stabilize the pressure to the predefined set point as mentioned above. In other examples, urine may be actively drained from the urinary bladder 112, e.g. using a pump.

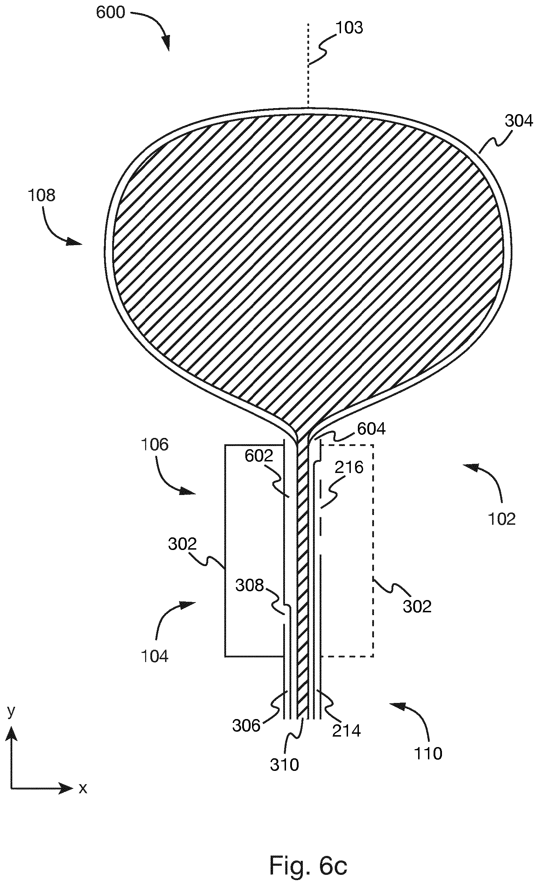

Figs. 6a-6c depict another example of a transurethral catheter device 600 according to an embodiment of the invention (not to scale). The catheter device 600 is similar to the catheter device 300 and also comprises an expandable body assembly 102 with two expandable bodies, namely a proximal inflatable balloon 302 and a distal inflatable balloon 304. In contrast to the catheter device 300, the proximal inflatable balloon 302 and the distal inflatable balloon 304 of the catheter device 600 are parts of independent catheter assemblies, wherein the distal inflatable balloon 304 is configured to be inserted through an insertion tube 602 extending through the proximal inflatable balloon 302 as detailed below. Fig. 6a schematically illustrates a sectional view of the catheter device 600 in a partially expanded state, in which the proximal inflatable balloon 302 is in an expanded state while the distal inflatable balloon 304 is in a collapsed state and is being inserted through the insertion tube 602. Fig. 6b shows a radial cross section 106r of the center portion 106 of the catheter device 600 in the expanded state, wherein the radial cross section 106r is the cross section in the x-z-plane perpendicular to the longitudinal axis 103, which may e.g. be aligned with the y-axis. Fig. 6c depicts the catheter device 600 in the expanded state. The expanded state of the catheter device 600 is the state in which both the proximal inflatable balloon 302 and the distal inflatable balloon 304 are expanded.

The proximal inflatable balloon 302 extends from the proximal portion 104 to the center portion 106. In the expanded state, a distal end of the proximal inflatable balloon 302 is directly adjacent to a proximal end of the distal inflatable balloon 304. The proximal inflatable balloon 302 may for example increase the rigidity of the center portion 106 and thereby may prevent excessive bending of the center portion 106.

In the example shown in Figs. 6a-6c, the proximal inflatable balloon 302 forms a partial cylinder in the expanded state, wherein the axis of the partial cylinder is aligned with the longitudinal axis 103. The proximal inflatable balloon 302 in particular forms a partial cylinder whose radial cross-section is a circular sector as shown in Fig. 6b. The circular sector may for example have a central angle between 210° and 330°, e.g. 270°. In other examples, the proximal inflatable balloon 302 may form a partial cylinder having a recess or cut-out with a different shape, e.g. a circular or rectangular cut-out. In some examples, the proximal inflatable balloon 302 may only be arranged in the proximal portion 104 and/or may have a different shape, e.g. an ellipsoidal shape, a cylindrical shape or a shape similar to the proximal inflatable balloon 302 of the catheter device 300 shown in Fig. 3. Furthermore, the catheter device 600 may comprise a central inflatable balloon (not shown) in addition to the proximal inflatable balloon 302, wherein the central inflatable balloon is arranged in the center portion 106 and may e.g. form a partial cylinder in the expanded state.

The proximal inflatable balloon 302 partially encloses the catheter tube assembly 110, which comprises the urine extraction tube 214 and the proximal inflation tube 306. The urine extraction tube 214 has a plurality of distal openings 216, which are located within the recess formed by the partial cylinder, i.e. within the circular sector that is not covered by the proximal inflatable balloon 302. In the example shown in Figs. 6a-6c, the catheter tube assembly 110 is located in the center of the proximal inflatable balloon 302. In other examples, the catheter tube assembly 110 may be located at a different position, e.g. in a cut-out adjacent to an outer edge of the proximal inflatable balloon 302.

The catheter tube assembly 110 further comprises the insertion tube 602, which extends through the proximal inflatable balloon 302, i.e. through the proximal portion 104 and the center portion 106. The insertion tube 602 comprises a distal opening 604, which is in communication with the inner volume of the urinary bladder 112 when the proximal inflatable balloon 302 is arranged in the urinary bladder 112. In some examples, the insertion tube 602 may be a trocar, i.e. may further comprise a removable obturator that is configured to block the distal opening 604 (not shown). An inner diameter of the insertion tube 602 is chosen such that the distal portion 108 of the expandable body assembly 102, i.e. the distal inflatable balloon 304, can be inserted through the insertion tube 602 in the collapsed state as shown in Fig. 6a.

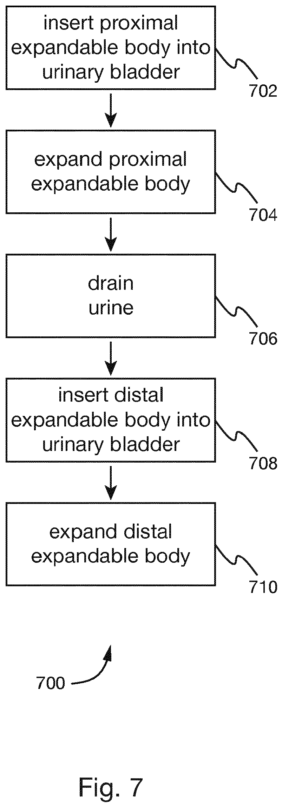

In the example of Figs. 6a-6c, the distal inflatable balloon 304 and the distal inflation tube 310 are not attached or connected to the other elements of the catheter device 600, i.e. the remaining elements of the catheter device 600 and the distal inflatable balloon 304 with the distal inflation tube 310 are independent catheter assemblies. This allows for first inserting the proximal inflatable balloon 302 into the urinary bladder 112 and expanding the proximal inflatable balloon 302, e.g. to provide access to the interior of the urinary bladder 112 via the urine extraction tube 214 similar to a conventional urinary catheter, and subsequently inserting and expanding the distal inflatable balloon 304 if needed. A method 700 for treating a patient with such a catheter device with two independent catheter assemblies is illustrated as a flowchart in Fig. 7.

At first, in step 702, the proximal expandable body, i.e. the proximal inflatable balloon 302, of the catheter device 600 is prepared in the collapsed state and is inserted into the urinary bladder 112 of the patient via the urethra 114, e.g. by pushing the proximal inflatable balloon 302 through the urethra 114 into the urinary bladder 112 using the catheter tube assembly 110.

Subsequently, in step 704, the proximal inflatable balloon 302 is expanded from the collapsed state to the expanded state such that the catheter device 600 is in the partially expanded state. This may prevent the proximal inflatable balloon 302 from entering the urethra 114 again and may thus secure the proximal inflatable balloon 302 within the urinary bladder 112. At this point, the catheter device 600 may be used similar to a conventional urinary catheter. The urine extraction tube 214 with the openings 216 is in communication with the inner volume of the urinary bladder 112 and may e.g. be used to drain urine from the urinary bladder 112 in 706.

Depending on the state of the patient, the distal expandable body, i.e. the distal inflatable balloon 304, may be inserted into the urinary bladder 112 in 708, either directly after step 702 or 704 or at a later point in time should the need arise. The distal inflatable balloon 304 may be inserted by threading the distal inflatable balloon 304 in the collapsed state through the insertion tube 602 using the distal inflation tube 310 as illustrated in Fig. 6a. After the distal inflatable balloon 304 has been arranged in the urinary bladder 112, the distal inflatable balloon 304 may be expanded as well in step 710 via the distal inflation tube 310, see Fig. 6c.

If the distal inflatable balloon 304 with the distal inflation tube 310 is an independent catheter assembly, the distal inflatable balloon 304 may also be removed independently from the other elements of the catheter device 600, e.g. after a bleeding has been stopped successfully. The proximal inflatable balloon 304 may for example remain in the urinary bladder 112 to provide the functionality of a conventional urinary catheter.

In other examples, the urine extraction tube 214 may be used as an insertion tube to insert the distal inflatable balloon 304 into the urinary bladder 112 (not shown). In this case, the distal opening 602 of the insertion tube may be one of the distal openings 216 of the urine extraction tube 214. Preferably, an outer diameter of the distal inflation tube 314 is less than 75%, in one example less than 50% of an inner diameter of the urine extraction tube 214, e.g. such as to not block the flow of urine through the urine extraction tube 214 when distal inflation tube 314 is arranged in the urine extraction tube 214. The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

- 100 -: transurethral catheter device
- 102 -: expandable body assembly
- 103 -: longitudinal axis
- 104 -: proximal portion of the expandable body assembly 102
- 106 -: center portion of the expandable body assembly 102
- 108 -: distal portion of the expandable body assembly 102
- 110 -: catheter tube assembly
- 112 -: urinary bladder
- 114 -: urethra
- 116 -: bottom portion of the wall of the urinary bladder 112
- 118 -: internal urethral orifice
- 120 -: upper portion of the wall of the urinary bladder 112
- 122 -: lateral portion of the wall of the urinary bladder 112
- 124 -: ureter orifices

- l₁ -: length of the proximal portion 104 in the expanded state
- w₁ -: width of the proximal portion 104 in the expanded state
- l₂ -: length of the center portion 106 in the expanded state
- w₂ -: width of the center portion 106 in the expanded state
- l₃ -: length of the distal portion 108 in the expanded state
- w₃ -: width of the distal portion 108 in the expanded state

- *l̃*₁ -: length of the proximal portion 104 in the collapsed state
- w̃₁ -: width of the proximal portion 104 in the collapsed state
- *l̃*₂ -: length of the center portion 106 in the collapsed state
- *w̃*₂ -: width of the center portion 106 in the collapsed state
- *l̃*₃ -: length of the distal portion 108 in the collapsed state
- *w̃*₃-: width of the distal portion 108 in the collapsed state

- 200 -: transurethral catheter device
- 202 -: inflatable balloon
- 204 -: wall of the inflatable balloon 202
- 206 -: inner volume of the inflatable balloon 202
- 208 -: constraining element
- 210 -: inflation tube
- 212 -: distal opening of the inflation tube 210
- 214 -: urine extraction tube
- 216 -: distal opening of the urine extraction tube 214

- 300 -: transurethral catheter device
- 304 -: distal inflatable balloon
- 302 -: proximal inflatable balloon
- 306 -: proximal inflation tube
- 308 -: distal opening of the proximal inflation tube 306
- 310 -: distal inflation tube
- 312 -: distal opening of the distal inflation tube 310

- 400 -: transurethral catheter device
- 402 -: urine reservoir
- 404 -: supply unit
- 406 -: pump
- 408 -: supply reservoir
- 410 -: control unit
- 412 -: flow meter
- 414 -: pressure sensor

- 500 -: method of using a transurethral catheter device
- 502 -: step of inserting the expandable body assembly 102 into the urinary bladder 112
- 504 -: step of expanding the expandable body assembly 102 to the expanded state
- 506 -: step of maintaining the displacement volume and/or pressure
- 508 -: step of draining urine from urinary bladder 112

- 600 -: transurethral catheter device
- 602 -: insertion tube
- 604 -: distal opening of the insertion tube 602
- 106r -: radial cross section of the center portion 106 of the transurethral catheter device 600

- 700 -: method of using a transurethral catheter device
- 702 -: step of inserting the proximal expandable body into the urinary bladder 112
- 704 -: step of expanding the proximal expandable body to the expanded state
- 706 -: step of draining urine from urinary bladder 112
- 708 -: step of inserting the distal expandable body into the urinary bladder 112
- 710 -: step of expanding the distal expandable body to the expanded state

## Claims

1. Transurethral catheter device (100) for bleeding control in pelvic fractures, the catheter device (100) comprising an expandable body assembly (102) with at least one expandable body, wherein
the expandable body assembly (102) comprises a proximal portion (104), a center portion (106) and a distal portion (108);
the expandable body assembly (102) is configured to be expanded from a collapsed state to an expanded state by expanding the at least one expandable body;
the expandable body assembly (102) is configured to be inserted into the urinary bladder (112) of a patient via the urethra (114) in the collapsed state;
in the expanded state, the proximal portion (104) has a length l₁ and a width w₁, the center portion (106) a length 12 and a width w2, the distal portion (108) a length l₃ and a width w₃, and the expandable body assembly (102) a total length 1;
a displacement volume V of the expandable body assembly (102) in the expanded state is larger than 0.5 liters;
the width w₂ is smaller than 50% of the width w₃;
the width w₁ is larger than a width *w̃*₁ of the proximal portion (104) in the collapsed state and the width w₃ is larger than a width *w̃*₃ of the distal portion (108) in the collapsed state; and
the length l₁ is smaller than 25% of the total length 1,
thereby allowing for arranging the expandable body assembly (102) in the expanded state in the urinary bladder (112) such that the proximal portion (104) is adjacent to the internal urethral orifice (118) of the urinary bladder (112) and the center portion (106) is adjacent to the ureter orifices (124) of the urinary bladder (112), but not in contact with the ureter orifices (124).

2. The catheter device (100) of claim 1, wherein
the displacement volume V of the expandable body assembly (102) in the expanded state is larger than 0.8 liters, preferably larger than 1.2 liters; and/or
the width w₂ is smaller than 20% of the width w₃; and/or
the width w₁ is at least two times as large as the width *w̃*₁; and/or
the length l₁ is smaller than 15% of the total length 1; and/or
the width w2 is smaller than 50% of the width w₁, preferably smaller than 30% of the width w₁.

3. The catheter device (100) of claim 1 or 2, wherein the catheter device (100) is configured to adjust the total length 1 such that, when the expandable body assembly (102) is arranged in the urinary bladder (112) in the expanded state, the proximal portion (104) is in contact with a bottom portion (116) of the wall of the urinary bladder (112) and the distal portion (108) is in contact with an upper portion (120) of the wall of the urinary bladder (112); and/or
wherein the catheter device (100) is configured to adjust the width w₃ such that, when the expandable body assembly (102) is arranged in the urinary bladder (112) in the expanded state, the distal portion (108) is in contact with a lateral portion (122) of the wall of the urinary bladder (112); and/or
wherein the catheter device (100) is configured to adjust the total length 1, the width w₁ and/or the width w₃ such that, when the expandable body assembly (102) is arranged in the urinary bladder (112) in the expanded state, the expandable body assembly (102) is in contact with at least 50%, preferably with at least 75% of a surface area of the wall of the urinary bladder (112).

4. The catheter device (100) of any one of the preceding claims, wherein the width w2 is smaller than 2 cm, preferably smaller than 1 cm, and/or wherein the length 12 is between 10% and 30% of the total length 1, and/or wherein the total length 1 is between 10 cm and 20 cm, preferably between 12.5 cm and 17.5 cm, and/or wherein the width w₃ is larger than, preferably at least twice as large as the width w₁.

5. The catheter device (100) of any one of the preceding claims, wherein an outer surface of the proximal portion (104) and/or an outer surface of the distal portion (108) has positive principal curvatures over at least 50%, preferably over at least 75% of the surface area of the respective portion in the expanded state; and/or wherein an outer surface of the center portion (106) has at least one principle curvature equal to or smaller than zero over at least 50%, preferably over at least 75% of the surface area of the center portion (106) in the expanded state; and/or wherein the expandable body assembly (102) is rotationally symmetric around a longitudinal axis extending from the proximal portion (104) to the distal portion (108).

6. The catheter device (200) of any one of the preceding claims, further comprising an urine extraction tube (214), wherein the urine extraction tube (214) has at least one distal opening (216) arranged in the center portion (106) of the expandable body assembly (102).

7. The catheter device (200) of any one of the preceding claims, wherein the expandable body assembly (102) comprises an expandable body extending from the proximal portion (104) to the distal portion (108).

8. The catheter device (200) of any one of the preceding claims, wherein the at least one expandable body comprises an inflatable balloon (202) comprising a flexible material (204) enclosing an inner volume (206) that is configured to receive an inflation medium, wherein the catheter device (200) preferably further comprises an inflation tube (210), wherein the inflatable balloon (202) is configured to receive the inflation medium through the inflation tube (210).

9. The catheter device (200) of claim 8, wherein the center portion (106) of the expandable body assembly (102) comprises at least one constraining element (208) configured to limit a width of the center portion (106).

10. The catheter device (200) of claim 8 or 9, wherein the inflation medium is a fluid.

11. The catheter device (400) of any one of claims 8 to 10, further comprising a volume control unit (410) configured to adjust the volume of the inflatable balloon (202) by adjusting a pressure in the inner volume (206) of the inflatable balloon (202) and/or by adjusting an amount of inflation medium supplied to the inner volume (206) of the inflatable balloon (202), wherein the volume control unit (410) preferably is configured to adjust the volume of the inflatable balloon (202) in the expanded state at least within a range between 1 liter and 1.5 liters, preferably at least within a range between 0.5 liters and 3 liters.

12. The catheter device (400) of any one of claims 8 to 11, further comprising a pressure sensor (414) configured to determine a pressure in the inner volume (406) of the inflatable balloon (202) and/or a pressure on an outer surface of the expandable body assembly (102).

13. The catheter device (300) of any one of the preceding claims, wherein the expandable body assembly (102) comprises a distal expandable body arranged in the distal portion (108) of the expandable body assembly (102) and a proximal expandable body arranged in the proximal portion (104) of the expandable body assembly (102).

14. The catheter device (300) of claim 13, wherein the proximal expandable body and the distal expandable body each comprise an inflatable balloon configured to receive an inflation medium, the catheter device (300) further comprising a proximal inflation tube (306) and a distal inflation tube (310), wherein the inflatable balloon (302) in the proximal portion (104) is configured to receive an inflation medium through the proximal inflation tube (306) and the inflatable balloon (304) in the distal portion (108) is configured to receive an inflation medium through the distal inflation tube (310).

15. The catheter device (600) of claim 13 or 14, further comprising an insertion tube (602) that extends through the proximal portion (104) and the center portion (106) of the expandable body assembly (102), wherein the distal portion (108) of the expandable body assembly (102) is configured to be inserted into the urinary bladder (112) through the insertion tube (602) in the collapsed state.

## Patentansprüche

1. Transurethrale Kathetervorrichtung (100) zur Blutungskontrolle bei Beckenfrakturen, wobei die Kathetervorrichtung (100) eine expandierbare Körperanordnung (102) mit mindestens einem expandierbaren Körper aufweist, wobei
die expandierbare Körperanordnung (102) einen proximalen Abschnitt (104), einen mittleren Abschnitt (106) und einen distalen Abschnitt (108) aufweist;
die expandierbare Körperanordnung (102) dazu eingerichtet ist, durch Expandieren des mindestens einen expandierbaren Körpers aus einem kollabierten Zustand in einen expandierten Zustand expandiert zu werden;
die expandierbare Körperanordnung (102) dazu eingerichtet ist, im kollabierten Zustand über die Harnröhre (114) in die Harnblase (112) eines Patienten eingeführt zu werden;
im expandierten Zustand der proximale Abschnitt (104) eine Länge l₁ und eine Breite w₁ aufweist, der mittlere Abschnitt (106) eine Länge l₂ und eine Breite w_{2,} der distale Abschnitt (108) eine Länge l₃ und eine Breite w₃ und die expandierbare Körperanordnung (102) eine Gesamtlänge 1;
ein Verdrängungsvolumen V der expandierbaren Körperanordnung (102) im expandierten Zustand größer als 0,5 Liter ist;
die Breite w₂ kleiner als 50% der Breite w₃ ist;
die Breite w₁ größer als eine Breite *w̃*₁ des proximalen Abschnitts (104) im kollabierten Zustand ist und die Breite w₃ größer als eine Breite *w̃*₃ des distalen Abschnitts (108) im kollabierten Zustand ist; und
die Länge l₁ kleiner als 25% der Gesamtlänge 1 ist,
wodurch die expandierbare Körperanordnung (102) im expandierten Zustand derart in der Harnblase (112) angeordnet werden kann, dass der proximale Abschnitt (104) benachbart zur inneren Harnröhrenöffnung (118) der Harnblase (112) ist und der mittlere Abschnitt (106) benachbart zu den Harnleiteröffnungen (124) der Harnblase (112) ist, aber nicht in Kontakt mit den Harnleiteröffnungen (124) ist;

2. Kathetervorrichtung (100) nach Anspruch 1, wobei
das Verdrängungsvolumen V der expandierbaren Körperanordnung (102) im expandierten Zustand größer als 0,8 Liter, vorzugsweise größer als 1,2 Liter ist; und/oder
die Breite w₂ kleiner als 20% der Breite w₃ ist; und/oder
die Breite w₁ mindestens zweimal so groß wie die Breite *w̃*₁ ist; und/oder
die Länge l₁ kleiner als 15% der Gesamtlänge 1 ist; und/oder
die Breite w₂ kleiner als 50% der Breite w₁, vorzugsweise kleiner als 30% der Breite w₁ ist.

3. Kathetervorrichtung (100) nach Anspruch 1 oder 2, wobei die Kathetervorrichtung (100) dazu eingerichtet ist, die Gesamtlänge 1 derart anzupassen, dass, wenn die expandierbare Körperanordnung (102) im expandierten Zustand in der Harnblase (112) angeordnet ist, der proximale Abschnitt (104) in Kontakt mit einem unteren Abschnitt (116) der Wand der Harnblase (112) ist und der distale Abschnitt (108) in Kontakt mit einem oberen Abschnitt (120) der Wand der Harnblase (112) ist; und/oder
wobei die Kathetervorrichtung (100) dazu eingerichtet ist, die Breite w₃ derart anzupassen, dass, wenn die expandierbare Körperanordnung (102) im expandierten Zustand in der Harnblase (112) angeordnet ist, der distale Abschnitt (108) in Kontakt mit einem lateralen Abschnitt (122) der Wand der Harnblase (112) ist; und/oder
wobei die Kathetervorrichtung (100) dazu eingerichtet ist, die Gesamtlänge l, die Breite w₁ und/oder die Breite w₃ derart anzupassen, dass, wenn die expandierbare Körperanordnung (102) im expandierten Zustand in der Harnblase (112) angeordnet ist, die expandierbare Körperanordnung (102) in Kontakt mit mindestens 50%, vorzugsweise mit mindestens 75% einer Oberfläche der Wand der Harnblase (112) ist.

4. Kathetervorrichtung (100) nach einem der vorhergehenden Ansprüche, wobei die Breite w2 kleiner als 2 cm, vorzugsweise kleiner als 1 cm ist, und/oder wobei die Länge l₂ zwischen 10% und 30% der Gesamtlänge l ist, und/oder wobei die Gesamtlänge l zwischen 10 cm und 20 cm, vorzugsweise zwischen 12,5 cm und 17,5 cm ist, und/oder wobei die Breite w₃ größer als die Breite w₁, vorzugsweise mindestens zweimal so groß wie die Breite w₁ ist.

5. Kathetervorrichtung (100) nach einem der vorhergehenden Ansprüche, wobei eine Au-βenfläche des proximalen Abschnitts (104) und/oder eine Außenfläche des distalen Abschnitts (108) positive Hauptkrümmungen über mindestens 50%, vorzugsweise über mindestens 75% der Oberfläche des jeweiligen Abschnitts im expandierten Zustand aufweist; und/oder wobei eine Außenfläche des mittleren Abschnitts (106) mindestens eine Hauptkrümmung gleich oder kleiner Null über mindestens 50%, vorzugsweise über mindestens 75% der Oberfläche des mittleren Abschnitts (106) im expandierten Zustand aufweist; und/oder wobei die expandierbare Körperanordnung (102) rotationssymmetrisch um eine Längsachse ist, die sich vom proximalen Abschnitt (104) zum distalen Abschnitt (108) erstreckt.

6. Kathetervorrichtung (200) nach einem der vorhergehenden Ansprüche, ferner aufweisend einen Urinextraktionsschlauch (214), wobei der Urinextraktionsschlauch (214) mindestens eine distale Öffnung (216) aufweist, die im mittleren Abschnitt (106) der expandierbaren Körperanordnung (102) angeordnet ist.

7. Kathetervorrichtung (200) nach einem der vorhergehenden Ansprüche, wobei die expandierbare Körperanordnung (102) einen expandierbaren Körper aufweist, der sich vom proximalen Abschnitt (104) zum distalen Abschnitt (108) erstreckt.

8. Kathetervorrichtung (200) nach einem der vorhergehenden Ansprüche, wobei der mindestens eine expandierbare Körper einen aufblasbaren Ballon (202) aufweist, der ein flexibles Material (204) aufweist, das ein Innenvolumen (206) umschließt, das dazu eingerichtet ist, ein Befüllungsmedium aufzunehmen, wobei die Kathetervorrichtung (200) vorzugsweise ferner einen Befüllungsschlauch (210) aufweist, wobei der aufblasbare Ballon (202) dazu eingerichtet ist, das Befüllungsmedium durch den Befüllungsschlauch (210) aufzunehmen.

9. Kathetervorrichtung (200) nach Anspruch 8, wobei der mittlere Abschnitt (106) der expandierbaren Körperanordnung (102) mindestens ein Beschränkungselement (208) aufweist, das dazu eingerichtet ist, eine Breite des mittleren Abschnitts (106) zu begrenzen.

10. Kathetervorrichtung (200) nach Anspruch 8 oder 9, wobei das Befüllungsmedium ein Fluid ist.

11. Kathetervorrichtung (400) nach einem der Ansprüche 8 bis 10, ferner aufweisend eine Volumensteuereinheit (410), die dazu eingerichtet ist, das Volumen des aufblasbaren Ballons (202) durch Anpassen eines Drucks im Innenvolumen (206) des aufblasbaren Ballons (202) und/oder durch Anpassen einer Menge an Befüllungsmedium, die dem Innenvolumen (206) des aufblasbaren Ballons (202) zugeführt wird, anzupassen, wobei die Volumensteuereinheit (410) vorzugsweise dazu eingerichtet ist, das Volumen des aufblasbaren Ballons (202) im expandierten Zustand mindestens innerhalb eines Bereichs zwischen 1 Liter und 1,5 Liter, vorzugsweise mindestens innerhalb eines Bereichs zwischen 0,5 Liter und 3 Liter, anzupassen.

12. Kathetervorrichtung (400) nach einem der Ansprüche 8 bis 11, ferner aufweisend einen Drucksensor (414), der dazu eingerichtet ist, einen Druck im Innenvolumen (406) des aufblasbaren Ballons (202) und/oder einen Druck auf einer Außenfläche der expandierbaren Körperanordnung (102) zu bestimmen.

13. Kathetervorrichtung (300) nach einem der vorhergehenden Ansprüche, wobei die expandierbare Körperanordnung (102) einen distalen expandierbaren Körper, der im distalen Abschnitt (108) der expandierbaren Körperanordnung (102) angeordnet ist, und einen proximalen expandierbaren Körper, der im proximalen Abschnitt (104) der expandierbaren Körperanordnung (102) angeordnet ist, aufweist.

14. Kathetervorrichtung (300) nach Anspruch 13, wobei der proximale expandierbare Körper und der distale expandierbare Körper jeweils einen aufblasbaren Ballon aufweisen, der dazu eingerichtet ist, ein Befüllungsmedium aufzunehmen, wobei die Kathetervorrichtung (300) ferner einen proximalen Befüllungsschlauch (306) und einen distalen Befüllungsschlauch (310) aufweist, wobei der aufblasbare Ballon (302) im proximalen Abschnitt (104) dazu eingerichtet ist, ein Befüllungsmedium durch den proximalen Befüllungsschlauch (306) aufzunehmen, und der aufblasbare Ballon (304) im distalen Abschnitt (108) dazu eingerichtet ist, ein Befüllungsmedium durch den distalen Befüllungsschlauch (310) aufzunehmen.

15. Kathetervorrichtung (600) nach Anspruch 13 oder 14, ferner aufweisend einen Einführschlauch (602), der sich durch den proximalen Abschnitt (104) und den mittleren Abschnitt (106) der expandierbaren Körperanordnung (102) erstreckt, wobei der distale Abschnitt (108) der expandierbaren Körperanordnung (102) dazu eingerichtet ist, im kollabierten Zustand durch den Einführschlauch (602) in die Harnblase (112) eingeführt zu werden.

## Revendications

1. Dispositif de cathéter transurétral (100) pour contrôle de saignement dans des fractures du bassin, le dispositif de cathéter (100) comprenant un ensemble corps déployable (102) avec au moins un corps déployable, dans lequel
l'ensemble corps déployable (102) comprend une partie proximale (104), une partie centrale (106) et une partie distale (108) ;
l'ensemble corps déployable (102) est configuré pour être déployé d'un état aplati à un état déployé en déployant l'au moins un corps déployable ;
l'ensemble corps déployable (102) est configuré pour être inséré dans la vessie (112) d'un patient via l'urètre (114) dans l'état aplati ;
dans l'état déployé, la partie proximale (104) a une longueur l₁ et une largeur w₁, la partie centrale (106) une longueur l₂ et une largeur w₂, la partie distale (108) une longueur l₃ et une largeur w₃ et l'ensemble corps déployable (102) une longueur totale l ;
un volume de déplacement V de l'ensemble corps déployable (102) dans l'état déployé est supérieur à 0,5 litre ;
la largeur w₂ est inférieure à 50 % de la largeur w₃ ;
la largeur w₁ est supérieure à une largeur *w̃*₁ de la partie proximale (104) dans l'état aplati et la largeur w₃ est supérieure à une largeur *w̃*₃ de la partie distale (108) dans l'état aplati ; et
la longueur l₁ est inférieure à 25 % de la longueur totale l,
permettant ainsi d'agencer l'ensemble corps déployable (102) dans l'état déployé dans la vessie (112) de telle sorte que la partie proximale (104) est adjacente à l'orifice urétral interne (118) de la vessie (112) et que la partie centrale (106) est adjacente aux orifices d'urètre (124) de la vessie (112), mais pas en contact avec les orifices d'urètre (124).

2. Dispositif de cathéter (100) selon la revendication 1, dans lequel
le volume de déplacement V de l'ensemble corps déployable (102) dans l'état déployé est supérieur à 0,8 litre, de préférence supérieur à 1,2 litre ; et/ou
la largeur w₂ est inférieure à 20 % de la largeur w₃ ; et/ou
la largeur w₁ est au moins deux fois supérieure à la largeur *w̃*₁ ; et/ou
la longueur l₁ est inférieure à 15 % de la longueur totale 1 ; et/ou
la largeur w₂ est inférieure à 50 % de la largeur w₁, de préférence inférieure à 30 % de la largeur w₁.

3. Dispositif de cathéter (100) selon la revendication 1 ou 2, dans lequel le dispositif de cathéter (100) est configuré pour ajuster la longueur totale 1 de telle sorte que, lorsque l'ensemble corps déployable (102) est agencé dans la vessie (112) dans l'état déployé, la partie proximale (104) est en contact avec une partie inférieure (116) de la paroi de la vessie (112) et la partie distale (108) est en contact avec une partie supérieure (120) de la paroi de la vessie (112) ; et/ou
dans lequel le dispositif de cathéter (100) est configuré pour ajuster la largeur w₃ de telle sorte que, lorsque l'ensemble corps déployable (102) est agencé dans la vessie (112) dans l'état déployé, la partie distale (108) est en contact avec une partie latérale (122) de la paroi de la vessie (112) ; et/ou
dans lequel le dispositif de cathéter (100) est configuré pour ajuster la longueur totale l, la largeur w₁ et/ou la largeur w₃ de telle sorte que, lorsque l'ensemble corps déployable (102) est agencé dans la vessie (112) dans l'état déployé, l'ensemble corps déployable (102) est en contact avec au moins 50 %, de préférence avec au moins 75 % d'une aire de surface de la paroi de la vessie (112).

4. Dispositif de cathéter (100) selon l'une quelconque des revendications précédentes, dans lequel la largeur w₂ est inférieure à 2 cm, de préférence inférieure à 1 cm, et/ou dans lequel la longueur l₂ est comprise entre 10 % et 30 % de la longueur totale l, et/ou dans lequel la longueur totale 1 est comprise entre 10 cm et 20 cm, de préférence entre 12,5 cm et 17,5 cm, et/ou dans lequel la largeur w₃ est supérieure à la largeur w₁, de préférence au moins deux fois supérieure.

5. Dispositif de cathéter (100) selon l'une quelconque des revendications précédentes, dans lequel une surface externe de la partie proximale (104) et/ou une surface externe de la partie distale (108) présente des courbures principales positives sur au moins 50 %, de préférence sur au moins 75 % de l'aire de surface de la partie respective dans l'état déployé ; et/ou dans lequel une surface externe de la partie centrale (106) présente au moins une courbure principale égale ou inférieure à zéro sur au moins 50 %, de préférence sur au moins 75 % de l'aire de surface de la partie centrale (106) dans l'état déployé ; et/ou dans lequel l'ensemble corps déployable (102) est symétrique en rotation autour d'un axe longitudinal s'étendant de la partie proximale (104) à la partie distale (108).

6. Dispositif de cathéter (200) selon l'une quelconque des revendications précédentes, comprenant en outre un tube d'extraction d'urine (214), dans lequel le tube d'extraction d'urine (214) présente au moins une ouverture distale (216) agencée dans la partie centrale (106) de l'ensemble corps déployable (102).

7. Dispositif de cathéter (200) selon l'une quelconque des revendications précédentes, dans lequel l'ensemble corps déployable (102) comprend un corps déployable s'étendant de la partie proximale (104) à la partie distale (108).

8. Dispositif de cathéter (200) selon l'une quelconque des revendications précédentes, dans lequel le au moins un corps déployable comprend un ballonnet gonflable (202) comprenant un matériau flexible (204) enfermant un volume interne (206) qui est configuré pour recevoir un milieu de gonflage, dans lequel le dispositif de cathéter (200) comprend de préférence en outre un tube de gonflage (210), dans lequel le ballonnet gonflable (202) est configuré pour recevoir le milieu de gonflage à travers le tube de gonflage (210).

9. Dispositif de cathéter (200) selon la revendication 8, dans lequel la partie centrale (106) de l'ensemble corps déployable (102) comprend au moins un agent de contrainte (208) configuré pour limiter une largeur de la partie centrale (106).

10. Dispositif de cathéter (200) selon la revendication 8 ou 9, dans lequel le milieu de gonflage est un fluide.

11. Dispositif de cathéter (400) selon l'une quelconque des revendications 8 à 10, comprenant en outre une unité de contrôle de volume (410) configurée pour ajuster le volume du ballonnet gonflable (202) en ajustant une pression dans le volume interne (206) du ballonnet gonflable (202) et/ou en ajustant une quantité de milieu de gonflage fourni au volume interne (206) du ballonnet gonflable (202), dans lequel l'unité de contrôle de volume (410) est de préférence configurée pour ajuster le volume du ballonnet gonflable (202) dans l'état déployé au moins dans une plage comprise entre 1 litre et 1,5 litre, de préférence au moins dans une plage comprise entre 0,5 litre et 3 litres.

12. Dispositif de cathéter (400) selon l'une quelconque des revendications 8 à 11, comprenant en outre un capteur de pression (414) configuré pour déterminer une pression dans le volume interne (406) du ballonnet gonflable (202) et/ou une pression sur une surface externe de l'ensemble corps déployable (102).

13. Dispositif de cathéter (300) selon l'une quelconque des revendications précédentes, dans lequel l'ensemble corps déployable (102) comprend un corps déployable distal agencé dans la partie distale (108) de l'ensemble corps déployable (102) et un corps déployable proximal agencé dans la partie proximale (104) de l'ensemble corps déployable (102).

14. Dispositif de cathéter (300) selon la revendication 13, dans lequel le corps déployable proximal et le corps déployable distal comprennent chacun un ballonnet gonflable configuré pour recevoir un milieu de gonflage, le dispositif de cathéter (300) comprenant en outre un tube de gonflage proximal (306) et un tube de gonflage distal (310), dans lequel le ballonnet gonflable (302) dans la partie proximale (104) est configuré pour recevoir un milieu de gonflage à travers le tube de gonflage proximal (306) et le ballonnet gonflable (304) dans la partie distale (108) est configuré pour recevoir un milieu de gonflage à travers le tube de gonflage distal (310).

15. Dispositif de cathéter (600) selon la revendication 13 ou 14, comprenant en outre un tube d'insertion (602) qui s'étend à travers la partie proximale (104) et la partie centrale (106) de l'ensemble corps déployable (102), dans lequel la partie distale (108) de l'ensemble corps déployable (102) est configurée pour être insérée dans la vessie (112) à travers le tube d'insertion (602) dans l'état aplati.
